# EUROPEAN PATENT SPECIFICATION

(11) **EP 0 781 756 B1**
(45) Date of publication and mention of the grant of the patent: **06.09.2000**
(21) Application number: 96308678.0
(22) Date of filing: 29.11.1996
(51) Int. Cl.: C07C 59/265, A23L 1/29, A61K 31/19, C07C 59/245

(54) **Soluble concentrated frozen/refrigerated calcium preparation comprising calcium citrate malate and process for producing the same**
Lösliches konzentriertes gefrorenes/gekühltes Calciumzitrat-Apfelsäuresalz enthaltende Calcium-Zusammensetzung und Verfahren zu ihrer Herstellung
Préparation concentrée soluble congélée/frigorifiée contenant du citrate et du sel d'acide maligne et procédé pour la préparation

(30) Priority: 28.12.1995 JP 34322695
(43) Date of publication of application: 02.07.1997
(73) Proprietor: Tanaka, Shiro, Shimizu-shi (JP); Tanaka, Shoichi, Shimizu-shi (JP); Tanaka, Miyoko, Shimizu-shi (JP)
(72) Inventor: Tanaka, Shiro, Shimizu-shi (JP); Hamano, Yoshihiko, Shimizu-shi (JP)
(74) Representative: Mallalieu, Catherine Louise

(56) References cited:
- EP-A- 0 297 679
- EP-A- 0 304 987
- GB-A- 2 103 089

## Description

The present invention relates to a process for producing a soluble concentrated frozen or refrigerated calcium preparation comprising calcium citrate.malate. More particularly but not exclusively it relates to a process for producing a soluble concentrated frozen or refrigerated calcium preparation which comprises reacting citric acid (or sodium citrate) and malic acid (or sodium malate) of prescribed proportions with a specified amount of calcium to obtain a soluble calcium preparation comprising calcium citrate.malate, and refrigerating or quickly freezing this solution to make inactive calcium in the solution and maintain its solubility permanently.

The soluble concentrated calcium preparation comprising calcium citrate.malate in accordance with the present invention can be used in any field where intake of calcium is needed, but is particularly useful in foods and pharmaceuticals.

### DESCRIPTION OF THE RELATED PRIOR ART

Supply of calcium which many Japanese tend to lack is one of the important projects of the Ministry of Health and Welfare, Japan. Also, with the rapid advance of an ageing society, a product capable of absorbing calcium efficiently in a solubilized state like calcium in milk is now an urgent global requirement. In view of this, some proposals on methods for solubilization of calcium have been made. For instance, Japanese Patent No. 1,836,036 discloses a method in which 70% or less of sodium citrate and 30% or more of sodium malate are dissolved in water, then a corresponding chemical molecular equivalent of calcium chloride is added to this solution and reacted, and the produced crystalline precipitate is separated and dried at a prescribed temperature. This method may be useful for solubilization of calcium salts which are normally slightly soluble. This method, however, is not suitable for the commercial supply of calcium preparations which can be widely used.

In view of the above, the present inventors have made extensive studies to find a simple method capable of supplying such soluble calcium preparations.

According to one aspect of the present invention there is provided a process for producing a soluble concentrated frozen or refrigerated calcium preparation comprising calcium citrate.malate, which comprises dissolving 90% or less of citric acid or sodium citrate and 10% or more of malic acid or sodium malate in water, adding thereto calcium of an amount of 1/2 of the sum of said acids or compounds, and refrigerating or quickly freezing the produced solution.

According to another aspect of the present invention there is provided a process for producing a soluble concentrated frozen or refrigerated calcium preparation comprising calcium citrate.malate, which comprises dissolving 90% or less of citric acid or sodium citrate and 10% or more of malic acid or sodium malate in water, adding thereto a corresponding chemical molecular equivalent of calcium, and refrigerating or quickly freezing the produced solution.

Preferably the % is weight % of the total weight of citric acid or sodium citrate and malic acid or sodium malate.

Preferably the % amount of citric acid or sodium citrate and malic acid or sodium malte is 100%.

Preferably half the amount by weight of calcium is added.

According to another aspect of the present invention there is provided a process for producing a soluble concentrated frozen or refrigerated calcium preparation comprising calcium citrate.malate, which comprises refrigerating or rapidly freezing a solution produced according to the process of the present invention, if necessary thawing out the refrigerated or frozen product, adding thereto a neutralization equivalent of calcium, stirring the solution until the reaction is completed, and then again freezing the solution.

Preferably the obtained frozen product is lyophilized in vacuo.

According to yet another aspect of the present invention there is provided a process for producing a soluble concentrated calcium preparation comprising calcium citrate.malate, which comprises thawing out a frozen product of the present invention, dehydrating the thawed product to a water content of about 50-60%, and subjecting it to tray type hot-air drying or spray drying to obtain a powdery preparation.

Preferably the water content is calculated with respect to that prior to drying.

Preferably the calcium used is calcium carbonate, calcium hydroxide, calcium chloride, calcium oxide or calcium lactate.

Preferably quick freezing is carried out at -18° to -50°C.

Preferably refrigeration is conducted at a temperature below +10°C, more preferably at +5° to -9°C.

We describe the use of the soluble concentrated calcium preparation comprising calcium citrate.malate in foods or pharmaceuticals.

Preferably the foods are drinks, dry products, confectionery, noodles or health foods.

Preferably the pharmaceuticals are injections or infusions.

Whilst not wishing to be bound by any theory the soluble calcium preparation comprising calcium citrate malate provided according to the present invention is assumed to have the following basic molecular structure (hydrogen bonds):

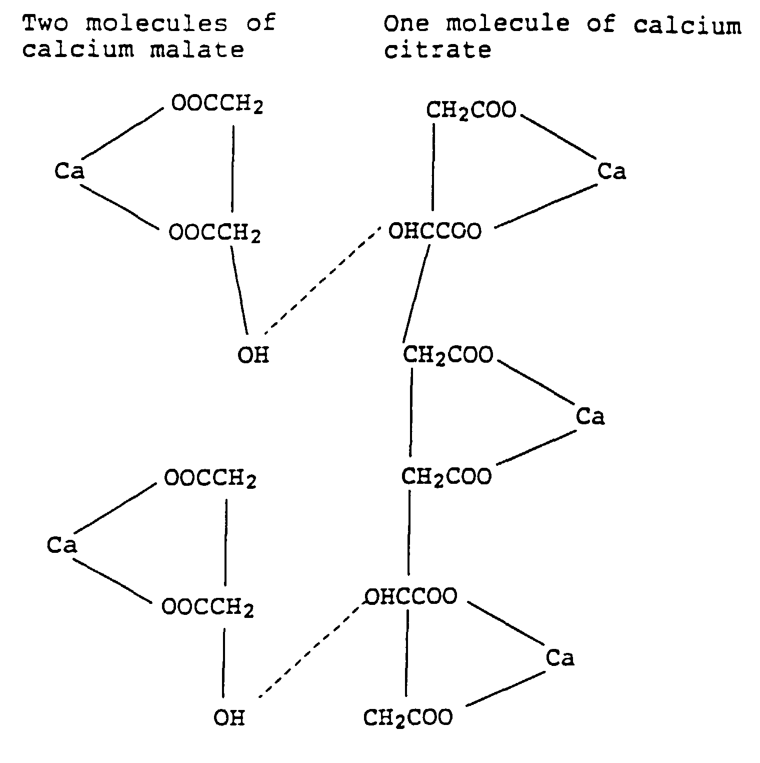

An increase of water solubility of calcium citrate, which is an organic acid in the Krebs cycle, is of great significance not only for the incorporation of calcium in foods and drinks but also for the use of calcium in pharmaceutics. It is particularly beneficial to drinks of the type which are required to be transparent at or around neutral pH. It is also advantageous in that addition of calcium to other foods can be made without fear of generating a calcium-like smell.

The following are examples of physical properties of preferred embodiments of this soluble calcium composition of the present invention.

| White crystal powder (neutral powder): Tasteless and odorless. 0.5 g of powder is soluble in 100 ml of 20°C water (pH = 6-6.5). composition: | | |
|---|---|---|
| | Theoretical | Found* |
| Calcium citrate | 42.24% | 40.48% |
| Calcium malate | 53.76% | 51.52% |
| Amount of calcium | 22.68% | 21.73% |
| Water | 4.0 % | 8.0% |

| White crystal fine powder with acid/calcium = 2/1 Sourness and odorless. 1.6 g of powder is soluble in 100 ml of 20°C water (pH = 3.9-4.0). | | |
|---|---|---|
| | Theoretical | Found* |
| Calcium citrate | 30.83% | 35.3% |
| Calcium malate | 39.05% | 47.7% |
| Amount of calcium | 16.51% | 16.0% |
| Water | 4.0% | 1.0% |

| Solution with acid/calcium = 2/1 Sourish and odoriferous although not unpleasant. 10.0 g is soluble in 100 ml of 20°C water (pH = 3.9-4.0). | | |
|---|---|---|
| Calcium citrate | 9.3% | 9.56% |
| Calcium malate | 11.8% | 13.4% |
| Amount of calcium | 5.0% | 5.0% |
| Water | 70.9% | 72.04% |

| | | |
|---|---|---|
| * According to the tests by Japan Canned Goods Testing Association. | | |

As mentioned above, as a result of the studies, the present inventors have succeeded in obtaining a soluble concentrated frozen or refrigerated calcium preparation by dissolving 99% or less of citric acid or sodium citrate and 10% or more of malic acid or sodium malate in water, adding thereto calcium of the amount of 1/2 of the sum of said acids or compounds, and refrigerating or quickly freezing the produced solution. It is a remarkable advantage of this preparation that it can be supplied either in a frozen state or if necessary as a dry product.

Various further preferred features and embodiments of the present invention will now be described by way of non-limiting example with reference to the accompanying Examples.

### Example 1

460 g of citric acid, 540 g of malic acid and 500 g of calcium carbonate were dissolved in 2.5 litres of water, and immediately thereafter the solution was quickly pan-frozen. 6 days later, the pan-frozen product was taken out of the refrigerator and thawed out by stirring with water of the amounts shown below. The resulting solution was then heated to 45-55°C, preferably 45±1°C. After this temperature had been reached, a corresponding chemical molecular equivalent of calcium carbonate was added, causing formation of a precipitate, and this precipitate was separated out and dried at 80-90°C to obtain the final product.

| | Initial blend | Neutralized blend | Deficit |
|---|---|---|---|
| Citric acid | 460 g | 460 g | 0 |
| Malic acid | 540 g | 540 g | 0 |
| Calcium carbonate | 500 g | 730 g | 230 g |
| Water | 2,500 ml | 4,110 ml | 1,610 ml |
| Total | 4,000 g | 5,840 ml | 1,840 ml |

In the initial blend: calcium carbonate/total = 12.5%; Ca content = 50 mg/g. In the neutralized blend: calcium carbonate/total = 12.5%; Ca content = 50 mg/g.
Results of the solubility test of the above product:
0.5%, pH = 5.96, soluble
0.45%, pH = 5.98, soluble
0.4%, pH = 6.02, soluble
0.30%, pH = 6.05, soluble

### Example 2

A soluble concentrated calcium preparation was produced according to the following formulation:

| | Initial blend | Neutralized blend | Deficit |
|---|---|---|---|
| 55°C hot water | 2,500 g | 4,110 g | 1,610 g |
| Citric acid | 460 g | 460 g | 0 |
| Malic acid | 540 g | 540 g | 0 |
| Calcium carbonate | 500 g | 730 g | 230 g |
| Total | 4,000 g | 5,840 g | 1,840 g |

### First method:

One-hour stirring after reaction → freezing → thawing the day after → supply of 1,610 g deficit of water → heating till reaching 55°C → supply of 230 g deficit of calcium carbonate → one-hour stirring → dehydration → disintegration and freezing→ lyophilizating the day after

### Results:

Water content = 4.9-5.2%.
Soluble at 0.5%, pH 5.8. Homogenization: 10,000 rpm/3 min.

### Second method:

Freezing immediately after reaction → thawing the day after → supply of 1,610 g deficit of 55°C hot water → heating to 45°C → supply of deficit of calcium carbonate → one-hour stirring → dehydration → disintegration → freezing → lyophilizating the day after.Results:
Water content = 2.9-5.0%.
Soluble at 0.5%, pH 5.83.

A soluble preparation is well obtainable from the first method, but the second method is considered a shade better. There is no difference from the case where the neutralization system was employed from the beginning, and the preparation is soluble at 0.5%.

### Example 3

Sample preparations obtained by using calcium carbonate (acid:calcium = 2:1) were subjected to a time test to obtain the following results.

| Normal temperature | | |
|---|---|---|
| Citric acid | malic acid | |
| 9 | 1 | Insoluble after preparation |
| 8 | 2 | Insoluble 3 days later |
| 7 | 3 | Insoluble 14 days later |
| 6 | 4 | Insoluble 18 days later |
| 5 | 5 | " |
| 4 | 6 | Insoluble 34 days later |
| 3 | 7 | Insoluble 24 days later |
| 2 | 8 | " |
| 1 | 9 | Insoluble 34 days later |

| 37°C | | |
|---|---|---|
| Citric acid | malic acid | |
| 8 | 2 | Insoluble one day later |
| 7 | 3 | " |
| 6 | 4 | " |
| 5 | 5 | " |
| 4 | 6 | " |
| 3 | 7 | " |
| 2 | 1 | " |
| 1 | 9 | " |

As is seen from the above, all the samples were insoluble. Since the sample with citric acid : malic acid = 9 : 1 was insoluble after preparation, it was not put into the thermostat.

### Example 4

Sample preparations obtained by using calcium hydroxide (acid : calcium = 2 : 1) were subjected to a time test to obtain the following results.

| Normal temperature | | |
|---|---|---|
| Citric acid | malic acid | |
| 9 | 1 | Insoluble after preparation |
| 8 | 2 | Insoluble 30 minutes later |
| 7 | 3 | Insoluble one day later |
| 6 | 4 | Insoluble 3 days later |
| 5 | 5 | " |
| 4 | 6 | Insoluble 5 days later |
| 3 | 7 | Insoluble 3 days later |
| 2 | 8 | " |
| 1 | 9 | Insoluble after preparation |

| 5°C | | |
|---|---|---|
| Citric acid | malic acid | |
| 6 | 4 | Insoluble 5 days later |
| 5 | 5 | " |
| 4 | 6 | " |
| 3 | 7 | " |
| 2 | 8 | " |

The samples with citric acid : malic acid = 9 : 1 and 1 : 9 were insoluble after preparation, and the sample with citric acid : malic acid = 7 : 3 became insoluble the day after preparation, so that the test was conducted on the samples which became insoluble thereafter.

| -18°C | | |
|---|---|---|
| Citric acid | malic acid | |
| 7 | 3 | Soluble |
| 6 | 4 | " |
| 2 | 8 | " |

The sample with citric acid : malic acid = 7 : 3 which became insoluble one day after preparation and the samples with citric acid : malic acid = 6 : 4 and 2 : 8 which became insoluble 3 days later were placed in a refrigerator and subjected to the test. As a result, calcium salt in these samples remained stable during the period when they were kept in the refrigerator.

### Example 5

Sample preparations obtained by using calcium oxide(acid:calcium = 2:1) were subjected to a time test to obtain the following results.

| Normal temperature | | |
|---|---|---|
| Citric acid | malic acid | |
| 9 | 1 | Insoluble immediately after preparation |
| 8 | 2 | " |
| 7 | 3 | Insoluble 2 days later |
| 6 | 4 | " |
| 5 | 5 | " |
| 4 | 6 | " |
| 3 | 7 | " |
| 2 | 8 | " |
| 1 | 9 | " |

| 5°C | | |
|---|---|---|
| Citric acid | malic acid | |
| 7 | 3 | Insoluble 6 days later |
| 6 | 4 | " |
| 5 | 5 | " |
| 4 | 6 | " |
| 3 | 7 | " |
| 2 | 8 | " |
| 1 | 9 | " |

The samples with citric acid : malic acid = 2 : 8 and 1 : 9 became insoluble distinctly. The samples with citric acid : malic acid = 8 : 2 and 9 : 1 were insoluble immediately after preparation, so that they were excluded from the test.

| -18°C | | |
|---|---|---|
| Citric acid | malic acid | |
| 6 | 4 | Soluble |
| 1 | 9 | " |

The sample with citric acid : malic acid = 1 : 9 which became insoluble distinctly and the sample with citric acid : malic acid = 6 : 4 which turned into a turbid insoluble state were subjected to a solubility test in which each sample was left in a refrigerator for 6 days and then thawed out. Calcium salt in the samples remained stable in the refrigerator throughout the test period.

### Example 6

A calcium preparation obtained from the following formulation according to the process of Example 1 was subjected to a comparative solubility test under the different conditions described below (acid:calcium = 2:1).

| Formulation | |
|---|---|
| Water | 125 g |
| Citric acid | 23 g |
| Malic acid | 27 g |
| Calcium carbonate | 25 g |
| Total | 200 g |

| Conditions | | |
|---|---|---|
| | | Calcium content |
| 1. | Normal temperature preparation (20-25°C) 8 days later, 2.5%, soluble | 125 mg/100 g |
| 2. | Refrigerated preparation (5°C) 8 days later, 3.0%, soluble | 150 mg/100 g |
| 3. | Frozen preparation (-18°C) 8 days later, 10.0%, soluble | 500 mg/100 g |
| 4. | Dried preparation (80°C) 1.6%, soluble | 240 mg/100 g |

As is seen from the above results, the frozen preparation maintains the same state as when it was prepared and does not cause activation of calcium salt, so that its solubility does not lower. The normal temperature preparation and the refrigerated preparation are slightly lowered in solubility, are still in accordance with the present invention. Since the dried preparation was dried under a severe temperature condition, its solubility (measured in terms of calcium content) is 1/2 of that of the frozen preparation but higher than those of the normal temperature preparation and the refrigerated preparation.

### Example 7

The sample preparations comprising sodium citrate, sodium malate and calcium lactate were subjected to a calcium solubility test. The Ca content in each sample was 50 mg/g. Also, each sample was soluble at 15 g/100 g.

The samples which were taken out of the freezer after 10-day freezing were also soluble at 15 g/100 g like just after preparation. Calcium lactate is soluble at 5 g/100 g (Ca content = 6.5 mg/g), but the frozen sample of this invention was soluble at 15 g/100 g (Ca content = 7.5 mg/g), showing an approximately 15% rise of solubility.

| | | | | | | | |
|---|---|---|---|---|---|---|---|
| Sodium citrate: | Sodium malate | | | | | | |
| 9 | 1 | Insolube after one-day freezing | | | | | |
| 8 | 2 | " 1.5 g/100 g | | | | | |
| 7 | 3 | " (0.75 mg/g) | | | | | |
| 6 | 4 | " | | | | | |

| | | 4 days later 15 g | 4 days later 10 g | 4 days later 9 g | 4 days later 3 g | 4 days later 2 g | 4 days later 1 g |
|---|---|---|---|---|---|---|---|
| 5 | 5 | Insoluble | Insoluble | - | - | - | Soluble |
| 4 | 6 | " | " | - | - | Soluble | - |
| 3 | 7 | " | " | - | Soluble | - | - |
| 2 | 8 | " | " | Soluble | - | - | - |
| 1 | 9 | " | " | " | - | - | - |

As is seen from the above results, solubility lowers conspicuously with time. It is particularly remarkable that the sample with the 5 : 5 ratio, which was soluble at 1.5 g/100 g in the morning, became insoluble 9 hours later. These results indicate that solubility of calcium in the above preparations is maintained stably when in a frozen state.

### Example 8

Each of the frozen calcium preparations of the following formulations was lyophilized and subjected to a solubility test. Each test specimen was prepared by freezing each calcium preparation at -20°C for one week and then lyophilizing it for 24 hours.

| Specimen 1 | |
|---|---|
| 55°C water | 330 g |
| Citric acid | 46 g |
| Malic acid | 54 g |
| Calcium carbonate | 56 g |
| Total | 486 g |
| Soluble at 0.5%. | |

| Specimen 2 | |
|---|---|
| Water | 259 g |
| Citric acid | 46 g |
| Malic acid | 54 g |
| Calcium oxide | 41 g |
| Total | 400 g |
| Insoluble at 1.4% and soluble at 1.3%. | |

| Specimen 3 | |
|---|---|
| Water | 370 g |
| Citric acid | 46 g |
| Malic acid | 54 g |
| Calcium chloride | 63 g |
| Total | 533 g |
| Insoluble at 1.7% and soluble at 1.6%. | |

### Example 9

In order to make sure that a sample with a (citric acid/malic acid) ratio which makes the composition insoluble in 30-40 minutes at normal temperature becomes able to maintain its solubility as a result of freezing and lyophilization, the following experiment was carried out.

A calcium solution (Ca content = 50 mg/g) was prepared from a formulation comprising 62.5 g of water, 22.5 g of citric acid, 2.5 g of malic acid and 12.5 g of calcium carbonate (citric acid : malic acid = 9 : 1). This solution was once frozen, then thawed out and subjected to a solubility test with the amount of the solution adjusted to 1.5 g (0.75 mg/g). It was confirmed consequently that the specimen was soluble.

The prepared solution was similarly frozen, lyophilized and subjected to a solubility test. The powdery product had a calcium content of 160 mg/g (found) and a water content of 3.6%. This powdery product was subjected to a solubility test as a preparation of 0.469 g (0.75 mg/g). It was soluble.

### Example 10

### Application of soluble calcium citrate·malate to pharmaceuticals:

Calcium ions tend to form scarcely soluble compounds with various kinds of anions, so that in many cases they can not safely be blended in liquid agents. Blending in injections and infusions is particularly difficult, so that the pharmaceutical manufacturers are taxing their ingenuity in blending calcium ions in pharmaceuticals. Use of citric acid for this purpose is commonly practiced. There are cases where the fluid is made acidic or a special salt is used.

A soluble concentrated frozen calcium preparation comprising calcium citrate·malate was put into an infusion (aqueous electrolyte and calory replenisher/retainer), sterilized in an autoclave at 120°C for 15 minutes and then subjected to a calcium precipitation test.

The test preparation was adjusted to a calcium content of 50 mg/g and kept in a refrigerator for 10 days. The main constituents of the infusion were dextrose, sodium chloride, calcium chloride and potassium glycerophosphate, pH of the infusion was 5.22 and its refractive index was 4.6%.

### ① Blend of 99.2 g of infusion and 0.8 g of the frozen preparation (Ca content = 40 mg/100 g)

The blend with a theoretical calcium content of 40 mg/100 g, rather high for an infusion, was sterilized in an autoclave at 120°C for 15 minutes. Result: No formation of precipitate took place. The blend was transparent and showed no difference from the original infusion. pH = 4.85.

### ② Blend of 99.8 g of infusion and 0.2 g of fine powder (Ca content = 42 mg/100 g)

The calcium content of the fine powder was 210 mg/g. The blend was sterilized in the same way as in the case of the blend ①.

Result: No formation of precipitate. The blend was of the same transparency as the original infusion. pH = 5.35.

### Example 11

680 g of sodium citrate, 800 g of sodium malate and 740 g of calcium chloride were dissolved in 1,780 g of water and immediately frozen quickly. 270 days thereafter, the frozen product was taken out of the freezer and allowed to thaw by itself (pH of the mother liquor = 5.92).

Using the thawed solution, about 0.7 *kin* of calcium-containing bread weighing 409 g and 15 cm high was made from the following recipe:

| Recipe | |
|---|---|
| Strong flour | 280.0 g |
| Sucrose | 17.0 g |
| Powdered skim milk | 6.0 g |
| Common salt* | 1.5 g |
| Butter | 11.0 g |
| Dry yeast | 3.0 g |
| Thawed solution** | 8.2 g |
| Water | 213.3 g |
| Total | 540.0 g |

| | |
|---|---|
| * Normally common salt is blended in an amount of 5 g, but the amount in the above recipe was used since the thawed solution produces salt from a reaction. | |
| ** Since the Ca content in the solution is 50 mg/g, 410 mg (50 mg x 8.2 g = 410 mg) of calcium is contained in about 0.7 *kin* of bread, which means that each piece of bread contains about 70 mg of calcium. One piece of this bread can well supply the calcium shortage for a day. | |

### Example 12

A powder preparation was made according to the process of Example 1 using calcium hydroxide in place of calcium carbonate, and a sports drink was produced using this powder preparation.

According to the following method, the quickly frozen product was thawed out, followed by addition of deficit water and calcium hydroxide for neutralization, and after necessary treatments, the solution was again frozen and dried.

| | Excess acid supply method | | Neutralization method |
|---|---|---|---|
| Water | 250 g | deficit 80 g | 330 g |
| Citric acid | 46 g | | 46 g |
| Malic acid | 54 g | | 54 g |
| Calcium hydroxide | 50g | deficit 6 g | 56 g |
| | 400 g | | 486 g |

Consequently, 120 g of a powder preparation could be obtained. Using this powder preparation, a sports drink was produced from the following recipe:

| Recipe | |
|---|---|
| Granulated sugar | 53.44 g |
| Fructose | 35.00 g |
| Citric acid | 3.00 g |
| Powder preparation | 4.50 g |
| Potassium chloride | 0.02 g |
| Vitamin C | 1.00 g |
| Refined salt | 1.00 g |
| Flavor | 2.00 g |
| Sodium bicarbonate | 0.02 g |
| Glycine | 0.02 g |
| Total | 100.00 g |

The above blend was dissolved in 5 litres of water and put into two 2.5-litre PET bottles (pH = 3.86; Bx = 6.08). The drink can be offered with various aromas by varying the flavor. The calcium content of the powder preparation was 210 mg/g. Since a cupful of the drink contains about 38 mg/g of calcium, two cups of the drink can supply the calcium shortage for a day. The powder preparation may be dispensed into the packs which are handy to carry. A person carrying the pack may cut it open, put the powder into water in a cup and dissolve it to prepare a drink whenever he wants to do so.

### Example 13

A rapidly frozen product of a solution of the following composition was thawed out, and a dressing (Italian style) was prepared using the defrozen solution.

| Composition | |
|---|---|
| Water | 250 g |
| Citric acid | 60 g |
| Malic acid | 40 g |
| Calcium oxide | 50 g |
| Total | 400 g |

| Dressing recipe | | |
|---|---|---|
| ① | Xanthane gum | 0.52 g |
| | Liquid fructose/dextrose | 34.00 g |
| | Sodium glutamate | 1.60 g |
| | Onion garlic powder | 4.00 g |
| | Spice mix | 7.20 g |
| | Roughly minced red pepper | 0.08 g |
| | Minced onion | 2.00 g |
| | Micronized black pepper | 0.40 g |
| | Water | 153.00 g |
| ② | Thawed solution | 30.00 g |
| | 50 GRN wine vinegar | 23.20 g |
| | Common salt | 108.00 g |
| ③ | Safflower oil | 28.00 g |
| | Oliver oil | 8.00 g |

The components ① were dissolved at 80°C for 15 minutes, followed by supply of the components ② and cooling to 30°C, and then the components ③ were added. The mixture was stirred and emulsified, kept under stirring and then put into two 200-ml bottles. There was thus obtained bottled dressing containing about 1,340 mg of calcium per bottle.

### Example 14

Using the calcium-containing thawed solution prepared in Example 10, a *kuzumochi* (pudding-like arrow root-starch cake) dessert was made from the following recipe:

| Recipe | |
|---|---|
| Water | 669.0 g |
| Isomerized sugar | 130.0 g |
| Soybean oligosaccharide | 70.0 g |
| White sugar | 15.0 g |
| Thickening polysaccharide | 11.0 g |
| Dextrin | 90.0 g |
| Thawed solution | 15.0 g |
| Total | 1,000 g |
| Bx = 27.0%; pH = 5.30. | |

There were obtained about 16 pieces of *kuzumochi* in the containers of 60 g, and they were sterilized by heating at 90°C for 10 minutes. The calcium content of *kuzumochi* was 45 mg/60 g.

### Example 15

Using the calcium-containing thawed solution used in Example 1, a fruit jelly was made from the following recipe:

| Recipe | |
|---|---|
| Water | 1,380.4 g |
| Defrozen solution | 40.0 g |
| Isomerized sugar | 260.0 g |
| White sugar | 260.0 g |
| Polysaccharide | 21.0 g |
| Apple juice | 34.0 g |
| Acidulant | 3.0 g |
| Flavor | 1.0 g |
| Color | 0.6 g |
| Total | 2,000.0 g |
| Bx = 25.0%; pH = 3.79. | |

The materials of the above specified amounts were packed in the containers of 120 g to form 16 pieces of jelly, and they were sterilized at 80°C for 10 minutes. The calcium content of the jelly was 120 mg/120 g, which shows that the calcium shortage for a day can well be supplied by taking one piece of jelly.

### Example 16

A solution of 15 g of sodium citrate, 15 g of sodium malate and 40 g of calcium lactate in 34 g of water was quickly frozen. 10 days thereafter, the frozen product was taken out of the freezer and allowed to thaw by itself (pH of the mother liquor = 7.20).

Using the thawed solution, calcium-containing *imoyokan* (sweet jelly of beans mixed with sweet potato) was made from the following recipe:

| Recipe | |
|---|---|
| Powdered agar | 0.6 g |
| Water | 50.0 g |
| Granulated sugar | 24.0 g |
| Isomerized sugar | 3.0 g |
| Thawed solution | 3.0 g |
| Roast sweet potato paste | 30.0 g |
| Total | 110.6 g |

The powdered agar was supplied into water and perfectly dissolved by boiling. Thereafter, granulated sugar was added and the solution was again boiled, followed by successive feed of the remaining materials. The mixed solution was heated till reaching 80°C, then packed in the containers, sterilized and cooled.

There were obtained 5 pieces of *imoyokan* in the one-kneck type 16 gram-portion containers. The calcium content of the mother liquor was 50 mg/g, and 3 g of this mother liquor was used. The jelly contained 22-26 mg of calcium per 16 g. Thus, 3 pieces of said jelly contain enough calcium to supply the calcium shortage for a day.

## Claims

1. A process for preparing a water-soluble frozen or refrigerated calcium composition comprising calcium citrate.malate, which comprises dissolving 90% or less of citric acid or sodium citrate and 10% or more of malic acid or sodium malate in an aqueous solution, adding calcium thereto in an amount which is half the amount of the sum of the amounts of said acids or salts, and refrigerating or freezing the resulting solution.

2. A process for preparing a water-soluble frozen or refrigerated calcium composition comprising calcium citrate.malate, which comprises dissolving 90% or less of citric acid or sodium citrate and 10% or more of malic acid or sodium malate in an aqueous solution, adding thereto a corresponding chemical molecular equivalent of calcium, and refrigerating or freezing the resulting solution.

3. A process according to claim 1 or claim 2 further comprising optionally thawing out the refrigerated or frozen composition adding to the resulting solution a neutralization equivalent of calcium and, after the reaction is completed, refrigerating or freezing the neutralised solution.

4. A process according to claim any one of claims 1-3, in which the solution is frozen and subsequently lyophilized in vacuo.

5. A process for preparing a water soluble calcium composition comprising calcium citrate.malate, which comprises thawing out a frozen composition prepared according to any one of claims 1 to 3, dehydrating the thawed product to a water content of about 50-60%, and subjecting it to drying to obtain a powdery preparation.

6. A process according to any one of claims 1 to 5, in which the calcium used is calcium carbonate, calcium hydroxide, calcium chloride, calcium oxide or calcium lactate.

7. A process according to any one of claims 1 to 6, in which freezing is carried out in the range of -18° to -50°C.

8. A process according to any one of claims 1 to 6, in which refrigeration or freezing is conducted at a temperature below +10°C.

9. A process according to claim 8, in which refrigeration or freezing is conducted at a temperature in the range of +5° to -9°C.

10. A process for preparing a food composition comprising admixing a calcium composition prepared according to the process of any one of claims 1 to 9 to a food.

11. A process for preparing a pharmaceutical composition comprising admixing a calcium composition prepared according to the process of any one of claims 1 to 9 to a pharmaceutical.

## Patentansprüche

1. Verfahren zur Herstellung einer gefrorenen oder gekühlten Calciumzusammensetzung, die Calciumcitrat-malat umfaßt, indem man 90% oder weniger Zitronensäure oder Natriumcitrat und 10% oder mehr Apfelsäure oder Natriummalat in einer wäßrigen Lösung auflöst, Calcium hierzu in einer Menge, welche die halbe Summe der Menge der Säuren oder Salze ist, zugibt und die resultierende Lösung kühlt oder gefriert.

2. Verfahren zur Herstellung einer wasserlöslichen gefrorenen oder gekühlten Calciumzusammensetzung, die Calciumcitrat-malat umfaßt, indem man 90% oder weniger Zitronensäure oder Natriumcitrat und 10% oder mehr Apfelsäure oder Natriummalat in einer wäßrigen Lösung auflöst, hierzu ein entsprechendes chemisches Molekularäquivalent von Calcium zugibt und die resultierende Lösung kühlt oder gefriert.

3. Verfahren nach Anspruch 1 oder Anspruch 2, bei dem man weiterhin gegebenenfalls die gekühlte oder gefrorene Zusammensetzung auftaut, zu der resultierenden Lösung ein Neutralisationsäquivalent von Calcium zugibt und nach Beendigung der Reaktion die neutralisierte Lösung kühlt oder gefriert.

4. Verfahren nach einem der Ansprüche 1 bis 3, bei dem die Lösung gefroren und anschließend im Vakuum gefriergetrocknet wird.

5. Verfahren zur Herstellung einer Calciumcitrat-malat umfassenden wasserlöslichen Calciumzusammensetzung, bei dem man eine nach einem der Ansprüche 1 bis 3 hergestellte gefrorene Zusammensetzung auftaut, das aufgetaute Produkt bis zu einem Wassergehalt von etwa 50 - 60% entwässert und es einer Trocknung unterzieht, um ein pulvriges Präparat zu erhalten.

6. Verfahren nach einem der Ansprüche 1 bis 5, bei dem das verwendete Calcium Calciumcarbonat, Calciumhydroxid, Calciumchlorid, Calciumoxid oder Calciumlactat ist.

7. Verfahren nach einem der Ansprüche 1 bis 6, bei dem das Gefrieren im Bereich von -18° bis -50°C durchgeführt wird.

8. Verfahren nach einem der Ansprüche 1 bis 6, bei dem das Kühlen oder Gefrieren bei einer Temperatur unterhalb +10°C durchgeführt wird.

9. Verfahren nach Anspruch 8, bei dem das Kühlen oder Gefrieren bei einer Temperatur im Bereich von +5° bis -9°C durchgeführt wird.

10. Verfahren zur Herstellung einer Nahrungsmittelzusammensetzung, bei dem man eine nach dem Verfahren nach einem der Ansprüche 1 bis 9 hergestellte Calciumzusammensetzung zu einem Nahrungsmittel zusetzt.

11. Verfahren zur Herstellung einer pharmazeutischen Zusammensetzung, bei dem man eine nach dem Verfahren nach einem der Ansprüche 1 bis 9 hergestellte Calciumzusammensetzung mit einem Arzneimittel vermischt.

## Revendications

1. Procédé de préparation d'une composition de calcium soluble dans l'eau, congelée ou réfrigérée, comprenant du citrate et du malate de calcium, comprenant les étapes consistant à dissoudre 90 % ou moins d'acide citrique ou de citrate de sodium et 10 % ou plus d'acide malique ou de malate de sodium dans une solution aqueuse, à y ajouter du calcium en une quantité qui vaut la moitié de la somme des quantités desdits acides ou sels et à réfrigérer ou à congeler la solution résultante.

2. Procédé de préparation d'une composition de calcium soluble dans l'eau, congelée ou réfrigérée, comprenant du citrate et du malate de calcium, comprenant les étapes consistant à dissoudre 90 % ou moins d'acide citrique ou de citrate de sodium et 10 % ou plus d'acide malique ou de malate de sodium dans une solution aqueuse, à y ajouter un équivalent moléculaire chimique correspondant de calcium et à réfrigérer ou à congeler la solution résultante.

3. Procédé selon la revendication 1 ou 2, comprenant en outre, le cas échéant, la décongélation de la composition réfrigérée ou congelée, l'addition de calcium en une quantité équivalente de neutralisation à la solution résultante, et quand la réaction est terminée, la réfrigération ou la congélation de la solution neutralisée.

4. Procédé selon l'une quelconque des revendications 1 à 3, dans lequel on congèle la solution et ensuite, on la lyophilise sous vide.

5. Procédé de préparation d'une composition de calcium soluble dans l'eau, comprenant du citrate et du malate de calcium, comprenant les étapes consistant à décongeler une composition congelée préparée selon l'une quelconque des revendications 1 à 3, à déshydrater le produit décongelé jusqu'à l'obtention d'une teneur en eau d'environ 50 à 60 % et à le soumettre à un séchage pour obtenir une préparation en poudre.

6. Procédé selon l'une quelconque des revendications 1 à 5, dans lequel le calcium utilisé est du carbonate de calcium, de l'hydroxyde de calcium, du chlorure de calcium, de l'oxyde de calcium ou du lactate de calcium.

7. Procédé selon l'une quelconque des revendications 1 à 6, dans lequel on effectue la congélation dans la plage de -18° à -50°C.

8. Procédé selon l'une quelconque des revendications 1 à 6, dans lequel on effectue la réfrigération ou la congélation à une température inférieure à +10°C.

9. Procédé selon la revendication 8, dans lequel on effectue la réfrigération ou la congélation à une température comprise entre +5° et -9°C.

10. Procédé de préparation d'une composition alimentaire, comprenant l'addition d'une composition de calcium préparée selon le procédé de l'une quelconque des revendications 1 à 9 à un aliment.

11. Procédé de préparation d'une composition pharmaceutique, comprenant l'addition d'une composition de calcium préparée selon le procédé de l'une quelconque des revendications 1 à 9 à une substance pharmaceutique.
